# FASCICULE DE BREVET EUROPEEN

(11) **EP 1 078 261 B1**
(45) Date de publication et mention de la délivrance du brevet: **27.10.2004**
(21) Numéro de dépôt: 99917007.9
(22) Date de dépôt: 07.05.1999
(51) Int. Cl.: G01N 33/543, G01N 33/547, C12N 11/06, C07C 235/20, C07D 495/04, C07C 233/40, C07F 1/00, C07F 15/04

(54) **PROCEDE DE FIXATION ET D'AUTO-ORGANISATION DE MACROMOLECULES BIOLOGIQUES SUR DES NANOTUBES DE CARBONE ET SES APPLICATIONS**
VERFAHREN ZUR FIXIERUNG UND SELBSTORGANISATION VON BIOLOGISCHEN MACROMOLEKULEN AUF KOHLENSTOFFNANORÖHREN UND DEREN VERWENDUNG
METHOD FOR IMMOBILISING AND SELF-ORGANIZING BIOLOGICAL MACROMOLECULES ON CARBON NANOTUBES AND USES

(30) Priorité: 07.05.1998 EP 98401114; 25.05.1998 FR 9806539
(43) Date de publication de la demande: 28.02.2001
(73) Titulaire: COMMISSARIAT A L'ENERGIE ATOMIQUE, 75015 Paris (FR); CENTRE NATIONAL DE LA RECHERCHE SCIENTIFIQUE (CNRS), 75794 Paris Cedex 16 (FR)
(72) Inventeur: BALAVOINE, Fabrice, F-75015 Paris (FR); MIOSKOWSKI, Charles, F-67200 Strasbourg (FR); SCHULTZ, Patrick, F-67640 Fegersheim (FR); RICHARD, Cyrille, F-92290 Chatenay-Malabry (FR)
(74) Mandataire: Goulard, Sophie
(86) Numéro de dépôt international: PCT/FR1999/001086
(87) Numéro de publication internationale: WO 1999/057564

(56) Documents cités:
- WO-A-97/32571
- TSANG SC ET AL: "Immobilization of small proteins in carbon nanotubes: high-resolution transmission electron microscopy study and catalytic activity" JOURNAL OF THE CHEMICAL SOCIETY, CHEMICAL COMMUNICATIONS, vol. 17, 1995, pages 1803-1804, XP002096861 cité dans la demande
- DAVIS JJ ET AL: "The immobilization of proteins in carbon nanotubes" INORGANICA CHIMICA ACTA, vol. 272, no. 1,2, 1998, pages 261-266, XP002096862 cité dans la demande
- FREY W ET AL: "Two-dimensional protein crystallization via metal-ion coordination by naturally occurring surface histidines" PROCEEDINGS OF THE NATIONAL ACADEMY OF SCIENCES OF USA, vol. 93, mai 1996 (1996-05), pages 4937-4941, XP002096863
- DIETRICH C, SCHMITT L, TAMP R: "Molecular organization of histidine-tagged biomolecules at self assembled lipid interfaces using a novel class of chelator lipids" PROCEEDINGS OF THE NATIONAL ACADEMY OF SCIENCES OF USA, vol. 92, septembre 1995 (1995-09), pages 9014-9018, XP002096864
- KUBALEK EW LE GRICE SFJ, BROWN PO: "Two-dimensional crystallization of histidine-tagged, HIV-1 reverse trascriptase promoted by a novel nickel chelating lipid" JOURNAL OF STRUCTUTRAL BIOLOGY, vol. 113, no. 2, 1994, pages 117-123, XP002096865 cité dans la demande

## Description

La présente invention est relative à un procédé de fixation et d'auto-organisation de macromolécules, aux réactifs chimiques mis en oeuvre dans ledit procédé, aux produits obtenus ainsi qu'aux applications desdits produits dans le domaine des matériaux et de la biologie structurale, notamment comme biocapteurs ou comme biomatériaux.

La connaissance de la structure des protéines et notamment de leurs sites actifs est essentielle à la compréhension de leur mécanisme d'action. On dispose, pour réaliser de telles études, de plusieurs méthodes : rayons X, RMN, électro-cristallographie (cristallisation 2D).

Pour réaliser la cristallisation proprement dite, la technique de cristallisation bidimensionnelle sur monocouche ou film lipidique, à l'interface air/eau (E.E. Ugziris et al., Nature, 1983, 301, 125-129), permet la formation de systèmes auto-organisés de macromolécules biologiques (cristaux) et la détermination des structures de ces molécules par l'analyse par microscopie électronique des cristaux obtenus.

Cette méthode consiste à créer une monocouche lipidique au niveau d'une interface air/liquide, les lipides étant sélectionnés pour interagir avec les protéines, présentes dans la phase liquide, qui se fixent sur les lipides, puis forment un réseau organisé.

La fixation des protéines sur les lipides de la monocouche met en jeu des interactions chimiques au niveau de la tête polaire des lipides. Ces interactions sont soit aspécifiques, les lipides possédant des extrémités polaires chargées, donnant lieu à une cristallisation par interactions ioniques, soit spécifiques. Dans ce dernier cas, la tête polaire des lipides porte des ligands présentant une forte affinité avec les protéines à fixer.

En particulier, il a pu être montré que des protéines solubles peuvent cristalliser bidimensionnellement sur des films lipidiques chargés, ou fonctionnalisés par un ligand de la protéine étudiée (B.J. Jap et al., Ultramicroscopy, 1992, 46, 45-84).

Plus récemment, des lipides fonctionnalisés par des complexes métalliques tels que des complexes de nickel (E.W. Kubalek et al., J. Struct. Biol., 1994, 113, 117-123) ont permis de cristalliser des protéines de fusion dites étiquetées histidine. Ces protéines possèdent en effet, à leur extrémité N- ou C-tenninale, une séquence composée de plusieurs histidines. Il a pu être montré que la fixation de telles protéines sur un lipide-nickel était due à une interaction forte entre le complexe nickel et la séquence poly-histidine (C. Vénien-Brian et al., J. Mol. Biot., 1997, 274, 687-692). De tels lipides fonctionnalisés ont permis d'obtenir une cristallisation, notamment dans les cas où l'on ne disposait pas du ligand approprié.

Toutefois, la cristallisation des protéines sur des films lipidiques présente l'inconvénient d'être relativement aléatoire et de dépendre de nombreux facteurs, qu'il est difficile de maîtriser simultanément :
- le ligand porté par les lipides doit être suffisamment accessible, pour pouvoir interagir avec les protéines. Cette accessibilité dépend de la longueur du bras espaceur entre le lipide et le ligand : trop court, il donne lieu à une pénétration de la protéine à l'intérieur de la couche lipidique ; trop long, il confère un trop grand degré de liberté à la protéine liée et augmente l'incidence des défauts dans le cristal ;
- la monocouche lipidique doit être suffisamment fluide pour conférer une mobilité latérale et rotationnelle suffisante à la protéine liée, permettant ainsi aux protéines de s'organiser les unes par rapport aux autres et de développer des contacts intermoléculaires, de façon à donner naissance au cristal ;
- une autre difficulté, inhérente à la cristallisation sur monocouche lipidique concerne la stabilité de la mono couche ; en effet, la stabilité de l'interface air/liquide est difficilement contrôlable. En outre, la monocouche lipidique doit rester stable, non seulement avant la fixation des protéines, mais aussi après leur fixation, pour permettre l'organisation spatiale des protéines ;
- pour l'étude microscopique, qui suit l'étape de cristallisation, il est nécessaire de réaliser une multitude de plans, du fait de la nature plane de la structure obtenue.

En conséquence, les Inventeurs se sont donné pour but de pourvoir à un procédé permettant de fixer en solution et d'induire une auto-organisation de macromolécules qui réponde mieux aux besoins de la pratique que les méthodes de cristallisation 2D antérieurement utilisées.

La présente invention a pour objet un procédé de fixation et d'auto-organisation de macromolécules biologiques, caractérisé en ce qu'il comprend l'incubation, sans agitation, pendant au moins 15 minutes, d'une macromolécule biologique en solution avec des nanotubes de carbone fermés à leurs extrémités, dans des conditions de température et de pH convenables.

Les nanotubes ont été découverts en 1991 (S. Ijima, *Nature* 1991, **354,** 54-56) ; depuis, ils ont suscité un très grand intérêt, notamment en raison de leurs propriétés mécaniques : grande résistance mécanique (M. M. J. Treacy *et al*., *Nature* 1996, **381,** pp. 678-680) et électroniques : propriété de conducteur ou de semiconducteur (J. W. G. Wildôer *et al*, *Nature* **1998,** 391, 59-62 ; T. W. Odom *et al*, *Nature,* 1998, **391,** 62-64).

Plusieurs procédés de préparation des nanotubes ont été décrits, dont celui de T. W. Ebbesen *et al*, *(Nature,* 1992, **358,** 220-222), qui permet d'obtenir un rendement élevé. Des méthodes de purification des nanotubes ont également été décrites (H. Hiura *et al*, *Adv. Mater.,* 1995, **7**, 275-276 ; J-M Bonard *et al*, *Adv. Mater*., 1997, **9**, 827-831 et G. S. Duesberg *et al*, *Chem. Commun*. **1998**, 435-436) ; ces différentes méthodes permettent d'obtenir les quantités souhaitées de nanotubes. Des méthodes de fonctionnalisation chimique des nanotubes de carbone ont également été décrites (Demande Internationale PCT WO 97/32571).

D'autres méthodes de fonctionnalisation chimique des nanotubes ont également été décrites ; on peut citer par exemple TSANG S.C. et al., *Journal of the Chemical Society, Chemical Communications*, 1995, **17**, 1803-1804 et DAVIS J.J. et al., *Inorganica Chimica Acta*, 1998, **272**, 1, 2, 261-266.

Toutefois, elles impliquent des réactions chimiques qui, soit modifient dramatiquement la géométrie des nanotubes (ouverture des extrémités, destruction partielle des feuillets externes), soit anéantissent les propriétés physiques intrinsèques des nanotubes et par conséquent ne permettent pas une organisation de macromolécules biologiques telles que les protéines, sur les nanotubes. Des nanotubes modifiés par de telles méthodes destructives ne sont donc pas adaptés à l'adsorption et/ou à l'auto-organisation à leur surface extérieure de produits synthétiques ou de macromolécules biologiques.

Selon la technique et les conditions employées, plusieurs structures de nanotubes peuvent être préparées : les nanotubes présentent notamment des structures dites en multi-feuillets (MWNT) ou en mono-feuillets (SWNT) de graphite. Ils peuvent être complètement, partiellement ou pas du tout oxydés.

Ainsi, les nanotubes sont, d'un point de vue chimique, des polymères composés uniquement de carbone et pouvant comporter jusqu'à un million d'atomes. Conformément aux lois de la chimie du carbone, les atomes d'un nanotube sont reliés par l'intermédiaire d'une solide liaison covalente et chaque atome possède exactement trois voisins. Ainsi quelle que soit sa longueur, un nanotube est obligé de se fermer à ses extrémités, de manière à n'y laisser aucune liaison chimique seule. Généralement son diamètre est généralement compris entre 1 et 30 nm et sa longueur peut atteindre plusieurs micromètres.

D'un point de vue physique, les nanotubes peuvent être définis comme des cristaux de carbone s'étendant dans une seule direction, le motif répétitif ayant la symétrie d'une hélice (B.I. Yakobson et al., American Scientist, 1997, 85, 324-337).

Selon un mode de mise en oeuvre avantageux dudit procédé, lesdites macromolécules biologiques sont notamment des protéines solubles, membranaires, trans-membranaires, des enzymes, des anticorps, des fragments d'anticorps ou des acides nucléiques.

Selon un autre mode de mise en oeuvre avantageux dudit procédé, lesdits nanotubes de carbone sont fonctionnalisés par adsorption physique d'un réactif chimique de formule générale **H-E-L**, dans laquelle :
- **H** représente un groupe hydrophile, sélectionné parmi les groupes chargés positivement ou négativement : des ligands ou analogues de macromolécules biologiques, tels que de manière non limitative, la biotine, la novobiocine. l'acide rétinoïque, les stéroïdes, des antigènes ; des complexes organométalliques interagissant avec des acides aminés ou des acides nucléiques, tels que les complexes de cuivre, de zinc, de nickel, de cobalt, de chrome, de platine, de palladium, de fer, de ruthénium ou d'osmium avec des ligands comme IDA, NTA, EDTA, bipyridine ou terpyridine, lesdits ligands étant éventuellement fonctionnalisés par des groupements alkyles de liaison à E (au niveau de X) ; on entend par groupes chargés positivement ou négativement et ce, de manière non limitative : ammoniums, carboxylates, phosphates, sulfonates ; on peut citer par exemple les groupes suivants : -N(CH₃)₃⁺ ou -CO₂⁻.
- **E** représente un bras espaceur, sélectionné parmi des chaînons carbonés en C₁-C₁₀, éventuellement substitués par des groupes alkyles ou non, présentant des insaturations ou des motifs poly-oxyéthylène pouvant présenter ou non en milieu de chaîne des groupes phosphate, tels que : 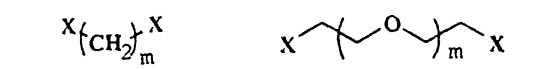 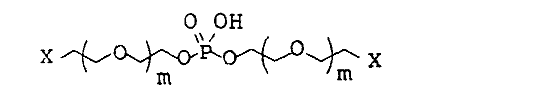 dans lesquels :
   m représente un nombre entier de 1 à 10,
   X représente O, NHCO, OCO, COO, CONH, S, CH₂ ou NH et constitue aux extrémités desdits chaînons carbonés des fonctions organiques d'accrochage du type esters, amides, éthers, thioéthers ;
- **L** représente un motif lipidique à une ou plusieurs chaînes de longueur variable, en C₁₂-C₂₀ présentant ou non des insaturations ; un groupe aromatique de formule Ar₁ ou de formule Ar₂ : 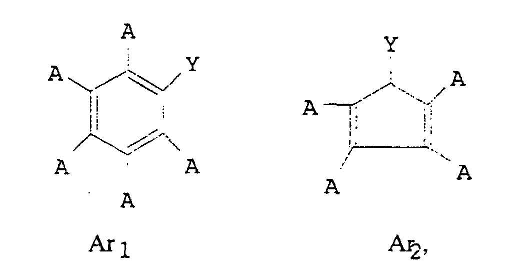 dans lesquelles :
   A représente un atome d'hydrogène, l'un des groupes suivants : alkyle, CF₃, NO₂, NH₂, OH, O-alkyle, S-alkyle, COOH, halogène, un cycle aromatique ou un hétérocycle aromatique en C₄-C₆, éventuellement polysubstitué par des groupes électrodonneurs de type alkyle ou électroattracteurs de type CF₃ ou halogénures ; L représente par exemple l'un des groupes aromatiques suivants : benzyle. naphtyle, anthracényle, fluorényle, tétrabenzofluorényle et
   Y représente une liaison à E.

On entend, au sens de la présente invention, par alkyle, des groupements alkyles en C₁-C₆, linéaires ou ramifiés ou éventuellement substitués.

De manière surprenante, aussi bien les nanotubes de carbone non traités (non fonctionnalisés) que les nanotubes de carbone fonctionnalisés par des méthodes non destructives, tels que définis ci-dessus, sont utilisables dans le procédé selon l'invention.

La fonctionnalisation selon la présente invention est, de manière surprenante, non destructive pour les nanotubes ; en particulier, elle évite l'ouverture de leurs extrémités.

Il est possible avec un tel procédé d'adsorber et/ou d'auto-organiser à la surface extérieure des nanotubes de carbone soit des produits synthétiques, soit des macromolécules biologiques.

En effet, la présente invention permet d'induire la formation d'arrangements de macromolécules (auto-organisation) telles que des protéines, avec une symétrie hélicoïdale.

) Selon un autre mode de mise en oeuvre dudit procédé, ladite solution est constituée d'un solvant de solubilisation desdites macromolécules biologiques, aqueux ou hydroalcoolique et contenant éventuellement au moins un détergent, en fonction de la macromolécule biologique à cristalliser.

Selon un autre mode de mise en oeuvre avantageux dudit procédé, les conditions d'incubation sont de préférence les suivantes : incubation à température ambiante, pendant 15 minutes à 48 heures, à un pH compris entre 5,5 et 8,5.

De manière surprenante, ledit procédé permet d'obtenir des arrangements de macromolécules biologiques permettant des études structurales par microscopie électronique et la préparation de nouveaux nano-matériaux utilisables pour leur propriétés physiques, électriques, ou biologiques.

Un tel procédé a l'avantage de rendre la cristallisation des protéines reproductibles ; en particulier, il est aisé, dans le cas où une protéine ne cristallise pas en présence de nanotubes d'un diamètre donné, de mettre en oeuvre le procédé avec des nanotubes de diamètre différent ; en effet, la cristallisation d'une protéine donnée est fonction du diamètre des nanotubes.

Or, dans la présente invention, il est possible de faire varier le diamètre des nanotubes et d'utiliser aussi bien des nanotubes de carbone multi-feuillets ou mono-feuillets.

Également de manière surprenante, dans le procédé selon l'invention, la fixation ou la cristallisation de macromolécules sur des nanotubes de carbone peut être, dans les conditions expérimentales appropriées, telles que définies ci-dessus, soit spontanées, c'est à dire en l'absence de tout autre produit synthétique, soit induites par addition d'un réactif chimique H-E-L, tel que défini ci-dessus.

Également de manière surprenante, les différents facteurs qui peuvent intervenir pour permettre une cristallisation reproductible sont, comme déjà précisés ci-dessus, les suivants : la concentration des échantillons, le choix des solvants, la force ionique, le pH des solutions, le temps d'incubation et le diamètre des nanotubes.

Aussi bien les réactifs dans lesquels **L** représente un motif lipidique à une ou plusieurs chaînes de longueur variable en C₁₂-C₂₀ présentant ou non des insaturations que les réactifs dans lesquels **L** représente un groupe aromatique de formule Ar₁ ou de formule Ar₂, permettent d'obtenir des nanotubes fonctionnalisés adaptés à l'arrangement de macromolécules à leur surface ; toutefois, les réactifs dans lesquels **L** représente un groupe aromatique de formule Ar₁ ou de formule Ar₂ sont particulièrement préférés.

La présente invention a également pour objet des bionanomatériaux, caractérisés en ce qu'ils sont constitués de nanotubes de carbone, sur lesquels des macromolécules biologiques sont auto-organisées sous une forme cristalline.

Selon un mode de réalisation avantageux desdits bionanomatériaux ils sont obtenus à l'aide d'un procédé tel que défini ci-dessus.

La présente invention a, en outre pour objet les applications desdits bionanomatériaux, à l'étude structurale des macromolécules biologiques qui leur sont associées, en tant que réactif biologique et plus particulièrement en tant que réactif immunologique et en tant que biocapteurs ou bioconducteurs.

La présente invention a, en outre, pour objet un réactif chimique apte à être adsorbé physiquement sur des nanotubes de carbone, caractérisé en ce qu'il présente la formule générale **H-E-L**, dans laquelle :
- **H** représente un groupe hydrophile, sélectionné parmi les groupes chargés positivement ou négativement ; des ligands ou analogues de macromolécules biologiques ; des complexes organométalliques interagissant avec des acides aminés ou des acides nucléiques et dont les ligands sont éventuellement fonctionnalisés par des groupements alkyles de liaison à **E** ;
- **E** représente un bras espaceur, sélectionné parmi des chaînons carbonés en C₁-C₁₀, éventuellement substitués par des groupes alkyles, présentant ou non des insaturations ou des motifs poly-oxyéthylène pouvant présenter ou non en milieu de chaîne des groupes phosphate, tels que : 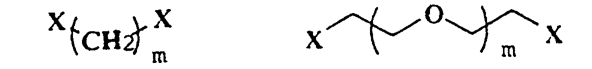 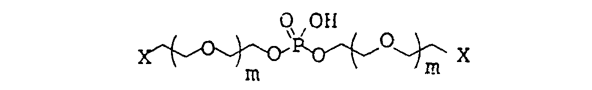 dans lesquels :
   m représente un nombre entier de 1 à 10,
   X représente O, NHCO, OCO, COO, CONH, S, CH₂ ou NH et constitue aux extrémités desdits chaînons carbonés des fonctions organiques d'accrochage du type esters, amides, éthers. thioéthers ;
- **L** représente un groupe aromatique de formule Ar₁ ou de formule Ar₂ : 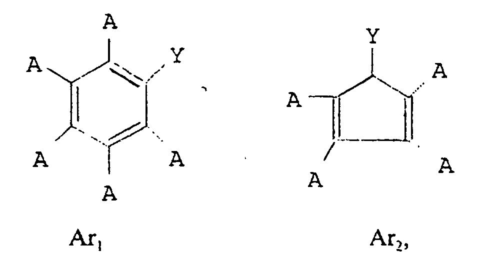 dans lesquelles :
   A représente un atome d'hydrogène, l'un des groupes suivants : alkyle, CF₃, NO₂, NH₂, OH, O-alkyle, S-alkyle, COOH, halogène, un cycle aromatique ou un hétérocycle aromatique en C₄-C₆, lesdits cycles étant éventuellement poly-substitués par des groupes électrodonneurs de type alkyle ou électroattracteurs de type CF₃ ou halogénures ; et
   Y représente une liaison à E.

Selon un mode de réalisation avantageux dudit réactif chimique, il présente l'une des structures suivantes :

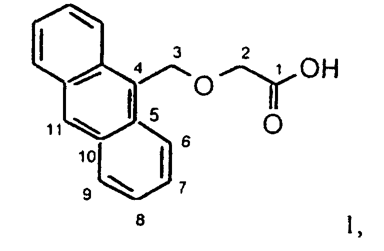

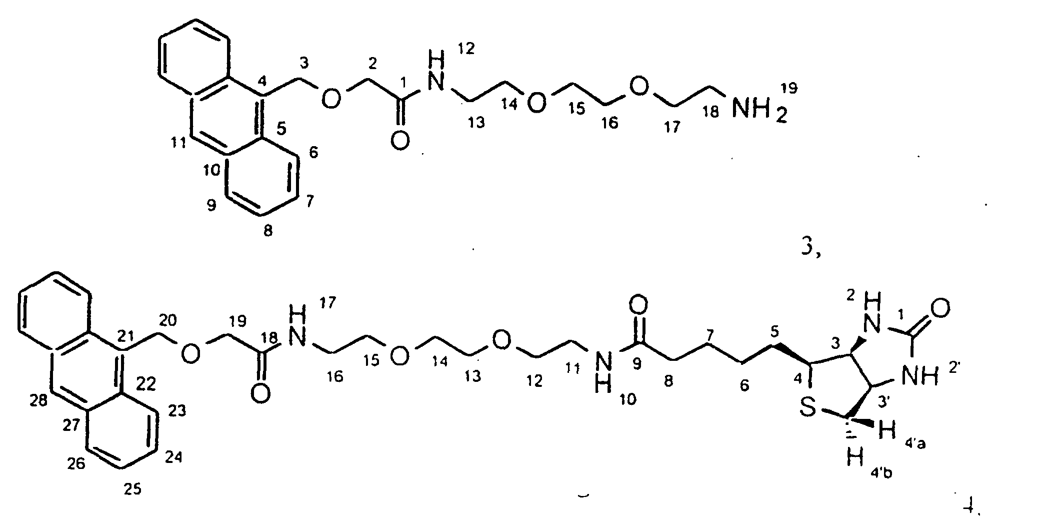

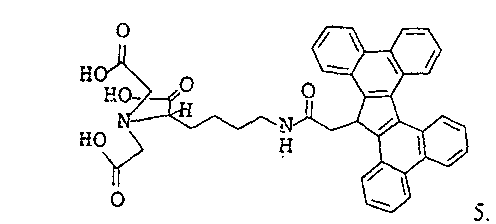

Selon un autre mode de réalisation avantageux dudit réactif chimique, H est sélectionné parmi les complexes organométalliques suivants : 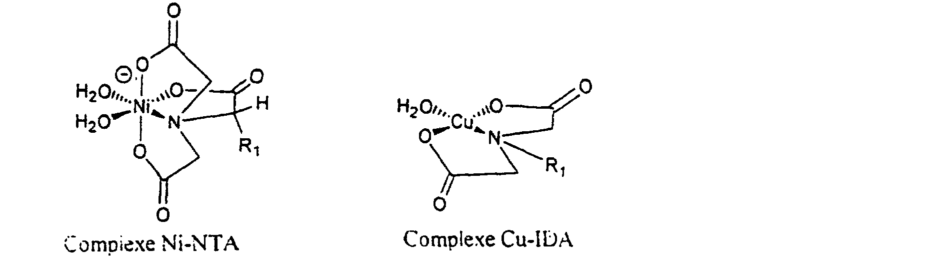 avec R₁ = groupe organique de liaison à E

Outre les dispositions qui précèdent, l'invention comprend encore d'autres dispositions, qui ressortiront de la description qui va suivre, qui se réfèrent à des exemples de mise en oeuvre du procédé objet de la présente invention ainsi qu'aux dessins annexés, dans lesquels :

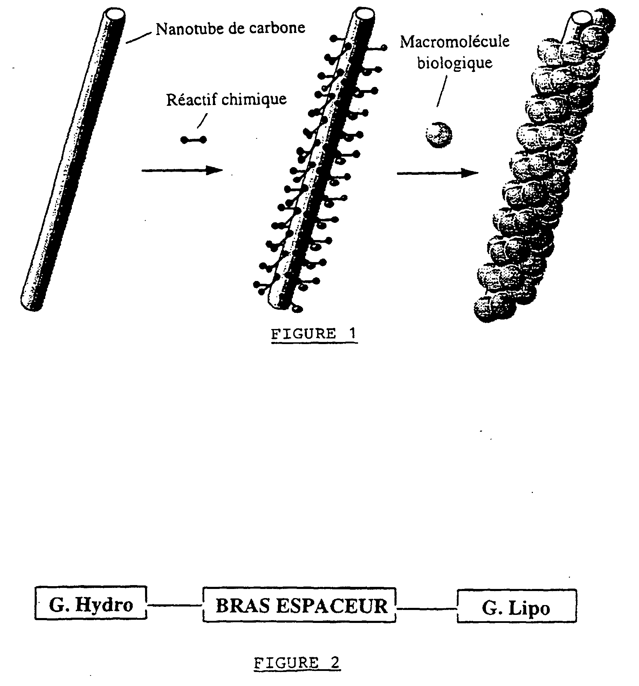

- la figure 1 illustre la cristallisation d'une macromolécule biologique sur un nanotube de carbone, par addition (adsorption physique) d'un réactif chimique ;
- la figure 2 illustre une structure de réactif chimique utilisé pour fonctionnaliser par adsorption physique les nanotubes de carbone ;

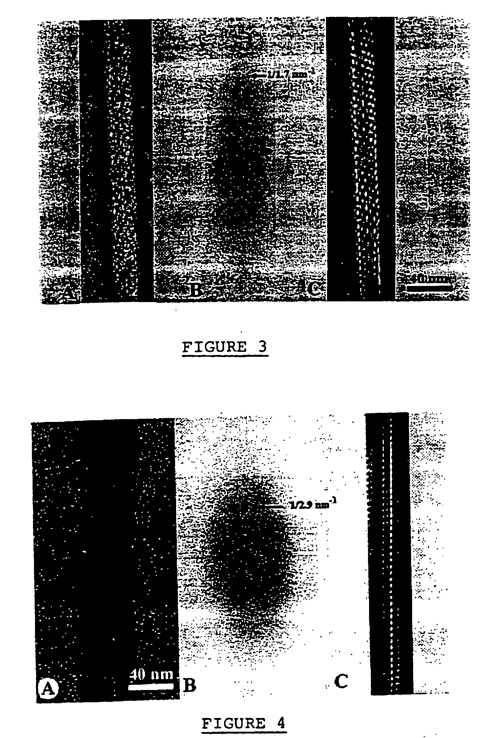

- la figure 3 représente des nanotubes d'un diamètre proche de 10 nm, couverts de cristaux hélicoïdaux de streptavidine ;
- la figure 4 représente des nanotubes couverts de cristaux hélicoïdaux de protéine HupR.

Il doit être bien entendu, toutefois, que ces exemples sont donnés uniquement à titre d'illustration objet de l'invention. dont ils ne constituent en aucune manière une limitation.

### Exemple 1 : Auto-organisation sur des nanotubes de carbone multi-feuillets de molécules de streptavidine en cristaux hélicoïdaux.

Les nanotubes de carbones multi-feuillets (MWNT) utilisés sont produits par décomposition d'une électrode de graphite par un arc-électrique (T. W. Ebbesen *et al*, *Nature* **1992**, vol 358, pp. 220-222). Après sonication d'une solution (2 mg/ml) de nanotubes de carbones, 20 µg de MWNT sont prélevés et séchés par un courant d'éthane-gaz pour être finalement remis en suspension dans 20 µl d'un mélange eau/méthanol (40% de méthanol en volume). Après sonication, 20 µl d'une solution aqueuse de streptavidine (10 µg/ml) sont additionnés et l'ensemble est laissé, sans agitation ni vortex, à température ambiante pendant 45 minutes. 5 µl de la suspension de nanotubes de carbone sont alors déposés sur une grille de microscopie électronique recouverte d'un film de carbone. Après une coloration négative de l'échantillon par une solution d'acétate d'uranyle, la grille est observée dans un microscope électronique (Philips CM 120). Il a pu être confirmé que les nanotubes d'un diamètre proche de 10 nm sont couverts de cristaux hélicoïdaux de streptavidine.

### Exemple 2 : Auto-organisation sur des nanotubes de carbone multi-feuillets de molécules de HupR "étiquetée-histidine" en cristaux hélicoïdaux.

Les nanotubes de carbones multi-feuillets (MWNT) utilisés sont produits par décomposition d'une électrode de graphite par un arc électrique (T. W. Ebbesen *et al*, *Nature* **1992**, vol 358, pp. 220-222). Après sonication d'une solution (2 mg/ml) de nanotubes de carbones, 20 µg de MWNT sont prélevés et séchés par un courant d'éthane gaz pour être finalement remis en suspension dans 20 µl d'un tampon aqueux (Tris 10 mM ; pH = 7,5 ; NaCl 350 mM). Après sonication, 20 µl d'une solution aqueuse de protéine HupR étiquetée-histidine (10 µg/ml) du *Rhodobacter Capsulatus* sont additionnés et l'ensemble est laissé, sans agitation ni vortex, à température ambiante pendant 25 minutes. 5 µl de la suspension de nanotubes de carbone sont alors déposés sur une grille de microscopie électronique recouverte d'un film de carbone. Après une coloration négative de l'échantillon par une solution d'acétate d'uranyle. la grille est observée dans un microscope électronique (Philips CM 120). Il a pu être noté qu'un grand nombre de nanotubes sont couverts de cristaux hélicoïdaux de protéine HupR.

### Exemple 3 : Préparation d'un réactif chimique biotinylé.

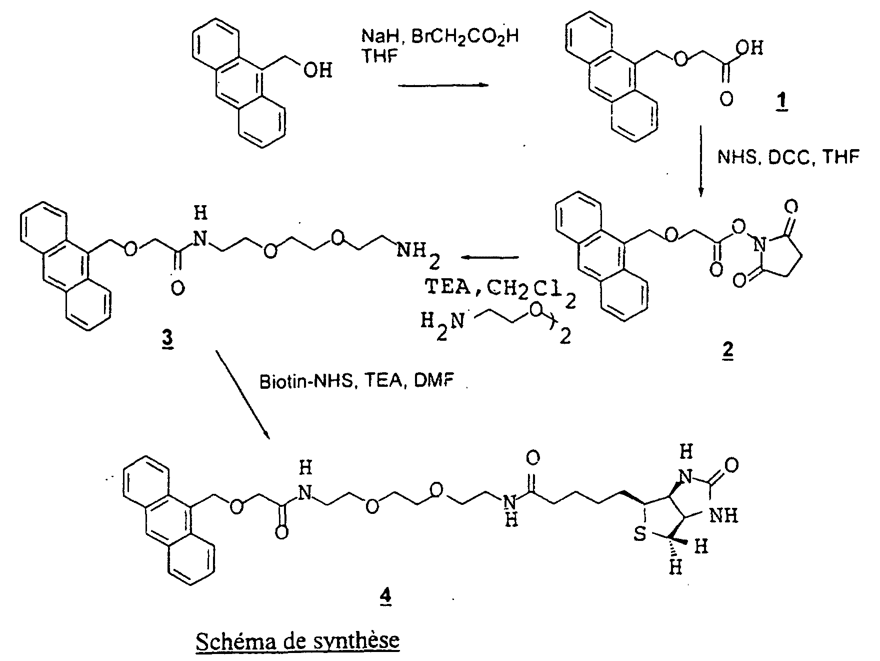

### Protocole expérimental

### Acide (anthracèn-9-ylméthoxy)-acétique 1 :

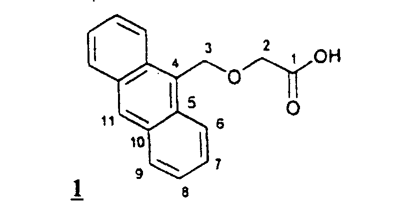

### MODE OPERATOIRE

A une suspension de 1,2 g (30 mmol, 3 éq.) d'hydrure de sodium à 60 % dans l'huile dans 20 ml de THF sont ajoutés à 0 °C 2,1 g (10 mmol, 1 éq) de 9-anthracènyl méthanol dans 20 ml de THF. Le mélange est agité pendant une heure à reflux, puis la température est abaissée à 0 °C pour ajouter 1,4 g (10 mmol, 1 éq.) d'acide bromoacétique en solution dans 20 ml de THF. La solution est agitée encore 5 minutes à 0 °C, puis une heure à température ambiante avant d'être portée à reflux pendant 16 heures. Après refroidissement. la réaction est stoppée par addition de 40 ml d'une solution aqueuse saturée en chlorure d'ammonium suivie de 10 ml d'une solution aqueuse d'acide chlorhydrique. Le milieu réactionnel est alors extrait avec 2 x 50 ml d'éther. Après séchage sur sulfate de sodium, la phase organique est évaporée à sec pour fournir après chromatographie sur silice (HexanelEtOAc/AcOH ; 70/30/1) 1,212 g d'un solide jaune (Rdt : 45,5 %).
**FB** : C₁₇H₁₄O₃ **PM** : 266,299 g/mol
CCM : Rf (EtOAc/Hex/AcOH ; 80120/1) : 0,56 ;
**RMN** ^{**1**}**H (300,13 MHz, Acétone d6)** : *δ* 11,2 (bs, 1H, H₁) ; 8,64 et 8,10 (d et d, 4H, J = 8,8 Hz, J = 7,9 Hz, H₆, H₉) ; 8,61 (s, 1H, H₁₁) ; 7,5 - 7,7 (m, 4H, H₇, H₈) ; 5,68 (s, 2H, H₃) ; 4,41 (s, 2H, H₂) ;
**RMN** ^{**13**}**C (75,47 MHz, Acétone d6)** : δ 176,27 (1C, C₁) ; 136,51 , 136,16 et 133,29 (5C, C₄, C₅, C₁₀) ; 133,75, 131,10 , 130,14 et 129,73 (9C, C₆, C₇, C₈, C₉, C₁₁) ; 71,88 (1C, C₃) ; 69,81 (1C, C₂) ;
**SM (70eV/DCI/intensité %)** : m/e : 191 (100, [M-OCH₂CO₂H]⁺) ; 266 (12, [M+1]⁺) ; 284 (58, [M+18]⁺) ;

### (Anthracèn-9-ylméthoxy)-acétate de 2,5-dioxo-pyrrolidin-1-yle 2 :

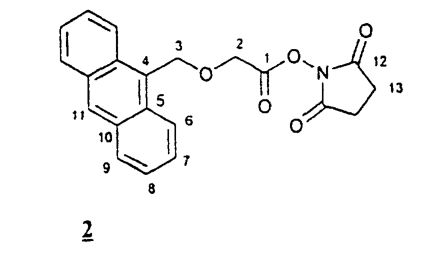

### MODE OPERATOIRE

A une solution de 650 mg (2,44 mmol, 1 éq) d'acide (anthracèn-9-ylméthoxy)-acétique **1** et 300 mg de NHS (2,61 mmol, 1,07 éq.) dans 30 ml de THF sont ajoutés à 0 °C 510 mg (2.47 mmol. 1 éq) de DCC en solution dans 20 ml de THF. Le mélange est agité pendant une nuit à température ambiante. Le milieu réactionnel est alors filtré, puis évaporé. Le résidu obtenu est repris dans 50 ml d'éthanol absolu pour fournir après filtration 727 mg d' (anthracèn-9-ylméthoxy)-acétate de 2.5-dioxo-pyrrolidin-1-yle **2** sous la forme d'un solide blanc (Rdt : 82%).
**FB** : C₂₁H₁₇NO₅ **PM** : 363,374 g/mol
CCM : Rf (EtOAc/Hex ; 50/50) : 0,33 ;
**RMN** ^{**1**}**H (300,13 MHz, CDCl**_{**3**}**)** : δ 8,51 (s, 1H, H₁₁) ; 8,42 et 8,02 (d et d, 4H, J = 9,0 Hz, J = 8,4 Hz, H₆, H₉) ; 7,59 et 7,48 (dd et dd, 4H, H₇, H₈) ; 5,71 (s, 2H, H₃) ; 4,53 (s, 2H, H₂) ; 2,90 (s, 4H, H₁₃) ;
**RMN** ^{**13**}**C (75,47 MHz, CDCl**_{**3**}**)** : **δ** 168,49 (2C, C₁₂) ; 166,16 (1C, C₁) ; 131,13 , 128,94 et 126,36 (5C, C₄, C₅, C₁₀) ; 128,82, 126,51 , 124,88 et 123,83 (9C, C₆, C₇, C₈, C₉, C₁₁) ; 65,28 (1C, C₃) ; 64,57 ( 1 C, C₂) ; 25,39 (2C, C₁₃) ;
**SM (70eV/DCI/intensité %)** : m/e : 381 (100, [M+18]⁺) ;

### N-{2-[2-(2-Amino-éthoxy)-éthoxy]-éthyl}-2-(anthracèn-9-ylméthoxy)-acétamide 3 :

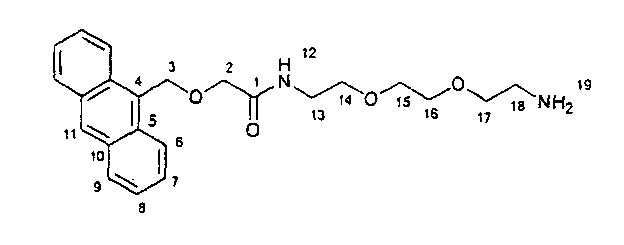

### MODE OPERATOIRE

A une solution de 370 mg (2.50 mmol, 10 éq.) de 2,2'-(éthylènedioxy)-diéthylamine dans 20 ml de CH₂Cl₂ sont ajoutés 1,75 ml de TEA et 91 mg (0,25 mmol, 1 éq) de (anthracèn-9-ylméthoxy)-acétate de 2,5-dioxo-pyrrolidin-1-yle **2** en solution dans 5 ml de CH₂Cl₂. Le mélange est agité pendant 7 heures à température ambiante. Le milieu réactionnel est alors évaporé. Le résidu obtenu est lavé avec 50 ml d'eau puis 50 ml d'une solution aqueuse d'hydroxyde de potassium (0,1 N). La phase organique est sechée, évaporée et concentrée sous vide pour fournir après chromatographie sur silice (CH₂Cl₂/MeOH/TEA ; 90/10/1). 55 mg de *N*-{2-[2-(2-Amino-éthoxy)-éthoxy]-éthyl}-2-(anthracèn-9-ylméthoxy)-acétamide **3** sous la forme d'une huile jaune (Rdt : 55%).
**FB** : C₂₃H₂₈N₂O₄ **PM** : 396.491 g/mol
**CCM** : Rf(CH₂Cl₂/MeOH/TEA ; 90/10/1) : 0,28 ;
**RMN** ^{**1**}**H (300,13 MHz, CDCl**_{**3**}**)** : δ 8,44 (s,1H, H₁₁) ; 8.29 et 7,98 (d et d. 4H, J = 9,0 Hz, J = 8,4 Hz, H₆, H₉) ; 7,53 et 7,45 (dd et dd, 4H, H₇, H₈) ; 6,93 (t, 1 H, J₆₋₉ = 5,4 Hz, 1H, H₁₂) ; 5,51 (s, 2H, H₃) ; 4,13 (s, 2H, H₂) ; 3,3 - 3,5 (m, 8H, H₁₄, H₁₅, H₁₆, H₁₇) ; 3,25 (t, J = 5,1 Hz, 2H, H₁₃) ; 2,7 - 2,8 (m, 2H, H₁₉) ; 2.64 (t, 2H, J = 5, 1 Hz,H₁₈) ;
**RMN** ^{**13**}**C (75,47 MHz, CDCl**_{**3**}**)** : δ 169,49 (1C, C₁) ; 131,11, 130,73 et 127,08 (5C, C₄, C₅, C₁₀) ; 128,92, 128,69 , 126,39 , 124,87 et 123,64 (9C, C₆, C₇, C₈, C₉, C₁₁) ; 72,10 , 69,89 , 69,78 , 69,47 et 69,33 (5C, C₃, C₁₄, C₁₅, C₁₆, C₁₇) ; 65,15 (1C, C₂) ; 41,03 (1C, C₁₃); 38,31 (1C, C₁₈) ;
**SM (70eV/DCI/intensité %)** : m/e : 397 (100, [M+1]⁺) ;

### N-{2-[2-(2-(Amino-biotine)-éthoxy)-éthoxy]-éthyl}-2-(anthracèn-9-ylméthoxy)-acétamide 4:

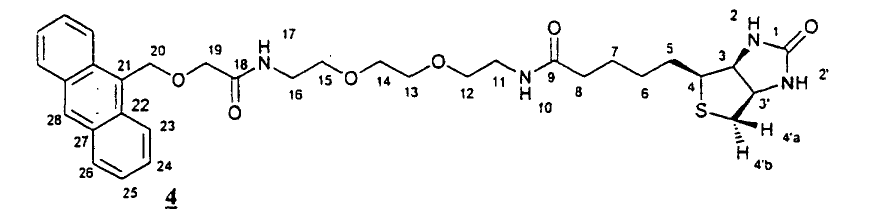

### MODE OPERATOIRE

A une solution de 40 mg (0,1 mmol, 1 éq.) de *N*-{2-[2-(2-amino-éthoxy)-éthoxy]-éthyl}-2-(anthracèn-9-ylméthoxy)-acétamide **3** dans 5 ml de DMF sont ajoutés 1 ml de TEA et 40 mg (0,12 mmol, 1,2 éq) de biotin-NHS en solution dans 5 ml de DMF. Le mélange est agité pendant 16 heures à température ambiante. Le milieu réactionnel est concentré sous vide pour fournir après chromatographie sur silice (CH₂Cl₂/MeOH/TEA ; 95/5/1); 55 mg de *N*-{2-[2-(2-(amino-biotine)-éthoxy)-éthoxy]-éthyl}-2-(anthracèn-9-ylméthoxy)-acétamide **4**: sous la forme d'une huile jaune (Rdt : 89%).
**FB** : C₃₃H₄₂N₄O₆S **PM** : 622,793 g/mol
**CCM** : Rf(CH₂Cl₂/MeOH ; 90/10) : 0.5 ;
**RMN** ^{**1**}**H (300,13 MHz, CDCl**_{**3**}**)** : δ 8,48 (s, 1H, H₂₈) ; 8,31 et 7,01 (d et d, 4H, J = 9,0 Hz, J = 8,4 Hz, H₂₃, H₂₆) ; 7,55 et 7,47 (dd et dd, 4H, H₂₄, H₂₅) ; 6,89 (t, 1H, J₁₆₋₁₇ = 5,0 Hz, H₁₇) ; 6,90 (s, 1H, H₂) ; 6,53 (t, 1H, J₁₀₋₁₁ = 5,0 Hz, H₁₀) ; 5,67 (s, 1H, H_{2'}) ; 5,55 (s, 2H, H₂₀) ; 4,31 (m, 1H, H3) ; 4,17 (s, 2H, H₁₉) ; 4,15 (m, 1H, H_{3'})†; 3,2 - 3,5 (m, 12H, H₁₁, H₁₂, H₁₃, H₁₄, H₁₅, H₁₆) ;2,97 (dt, 2H, H₄) ; 2,75 (dt, 1H, J_{3'-4'a} = 4,8 Hz, J_{4'a-4'b} = 12,7 Hz, H_{4'a}) ; 2,2 (d, 1H, J_{4'a-4'b} = 12,7 Hz, H_{4'b}) ; 2,08 (t, 2H, J₇₋₈ = 7,4 Hz, Hg) ; 1,2 - 1,7 (m, 6H, H₅, H₆, H₇) ;
**RMN** ^{**13**}**C (75,47 MHz, CDCl**_{**3**}**)** : δ 173,00 (1C, C₁) ; 169,56 (1C, C₁₈) ; 163,76 (1C, C₉) ; 131,14 , 130,73 et 127,05 (5C, C₂₁, C₂₂, C₂₇) ; 128,98, 128,75 , 126,45 , 124,93 et 123,59 (9C, C₂₃, C₂₄, C₂₅, C₂₆, C₂₈) ; 69,74 , 69,55 , 69.23 et 65,25 (6C, C₁₂, C₁₃, C₁₄, C₁₅, C₁₉, C₂₀) ; 61,50 et 59,88 (2C, C₃, C_{3'}) ; 55,32 (1C, C₄) ; 40,18 (1C, C_{4'}) ; 38,76 , 38,33 et 35,65 (3C, C₈, C₁₁, C₁₆) ; 27,95 et 27,81 (2C, C₅, C₇) ; 25,30 (1C, C₆);
**SM (70eV/DCI/intensité %)** : m/e : 623 (100, [M+1]⁺) ; 640 (8, [M+18]⁺) ;

### Exemple 4 : Adsorption physique sur des nanotubes de carbone d'un réactif chimique, dénommé ci-après CR174, de structure H-E-L

Protocole : A 20 µl d'une solution de nanotubes de carbone (10 mg/ml dans le méthanol), fraîchement soniquée, sont ajoutés 1 à 20 µl d'une solution de réactif chimique, dénommé CR174 et dont la formule chimique est illustrée ci-après, (1mg/ml) dans du méthanol. Le mélange est alors agité par sonication puis évaporé à sec par un courant d'éthane gaz, 40 µl de tampon Tris (20 mM, pH 7,5 ; NaCl 50 mM) sont ajoutés aux nanotubes de carbone secs et la suspension est remélangé par sonication. La suspension peut éventuellement être centrifugée et lavée plusieurs fois avec 500 µl de tampon pour éliminer l'exces de réactif non-adsorbé sur les nanotubes de carbone.

L'adsorption physique du réactif CR174 sur les nanotubes de carbone a pu être mise en évidence par microscopie électronique en coloration positive. La présence de molécules de réactifs sur les nanotubes de carbone se traduit par l'apparition de points noirs. Ces points noirs sont absents en l'absence de réactif chimique CR174 (ou 5).

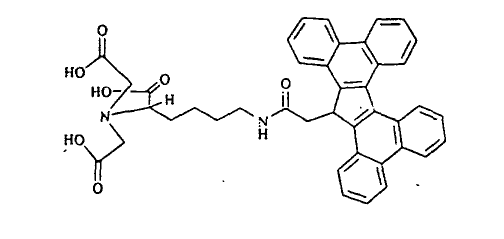

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Procédé de fixation et d'auto-organisation de macromolécules biologiques, **caractérisé en ce qu'**il comprend l'incubation, sans agitation, pendant au moins 15 minutes, d'une macromolécule biologique en solution avec des nanotubes de carbone fermés à leurs extrémités, dans des conditions de température et de pH convenables.

2. Procédé selon la revendication 1, **caractérisé en ce que** lesdites macromolécules biologiques sont notamment des protéines solubles, membranaires, trans-membranaires, des enzymes, des anticorps, des fragments d'anticorps ou des acides nucléiques.

3. Procédé selon la revendication 1 ou la revendication 2, **caractérisé en ce que** lesdits nanotubes de carbone sont fonctionnalisés par adsorption physique, à leur surface, d'un réactif chimique de formule générale **H-E-L**,
dans laquelle:
- **H** représente un groupe hydrophile, sélectionné parmi les groupes chargés positivement ou négativement ; des ligands ou analogues de macromolécules biologiques ; des complexes organométalliques interagissant avec des acides aminés ou des acides nucléiques et dont les ligands sont éventuellement fonctionnalisés par des groupements alkyles de liaison à E ;
- **E** représente un bras espaceur, sélectionné parmi des chaînons carbonés en C₁-C₁₀, éventuellement substitués par des groupes alkyles, présentant ou non des insaturations ou des motifs poly-oxyéthylène pouvant présenter ou non en milieu de chaîne des groupes phosphate, tels que : 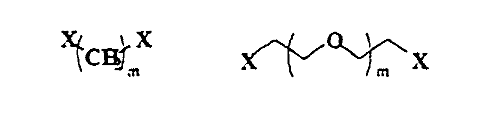 dans lesquels :
m représente un nombre entier compris entre 1 et 10, 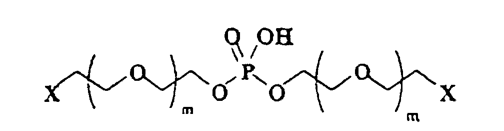 **X** représente O, NHCO, OCO, COO, CONH, S, CH₂ ou NH et constitue aux extrémités desdits chaînons carbonés des fonctions organiques d'accrochage du type esters, amides, éthers, thioéthers ;
- **L** représente un motif lipidique à une ou plusieurs chaînes de longueur variable, en C₁₂-C₂₀ présentant ou non des insaturations ; un groupe aromatique de formule Ar₁ ou de formule Ar₂ : 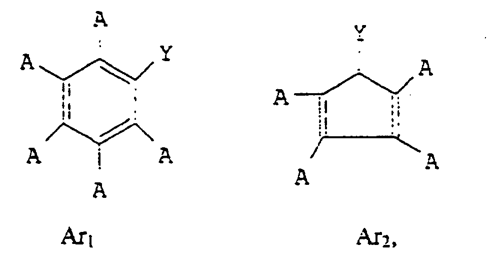 dans lesquelles :
A représente un atome d'hydrogène, l'un des groupes suivants : alkyle, CF₃, NO₂, NH₂, OH, O-alkyle, S-alkyle, COOH, halogène, un cycle aromatique ou un hétérocycle aromatique en C₄-C₆, éventuellement polysubstitué par des groupes électrodonneurs de type alkyle ou électroattracteurs de type CF₃ ou halogénures ; et
Y représente une liaison à E.

4. Procédé selon l'une quelconque des revendications 1 à 3, **caractérisé en ce que** ladite solution est constituée d'un solvant de solubilisation desdites macromolécules biologiques, aqueux ou hydroalcoolique et contenant éventuellement au moins un détergent.

5. Procédé selon l'une quelconque des revendications 1 à 4, **caractérisé en ce que** les conditions d'incubation sont de préférence les suivantes : incubation à température ambiante, pendant 15 minutes à 48 heures, à un pH compris entre 5,5 et 8,5.

6. Bionanomatzriaux, **caractérisés en ce qu'**ils sont constitués de nanotubes de carbone, sur lesquels des macromolécules biologiques sont auto-organisées sous une forme cristalline.

7. Bionanomatériaux selon la revendication 6, **caractérisés en ce qu'**ils sont obtenus à l'aide d'un procédé selon l'une quelconque des revendications 1 à 5.

8. Utilisation des bionanomatériaux selon l'une quelconque des revendications 6 à 7, pour l'étude structurale des macromolécules biologiques qui leur sont associées.

9. Utilisation des bionanomatériaux selon l'une quelconque des revendications 6 à 7, en tant que réactif biologique.

10. Utilisation des bionanomatériaux selon l'une quelconque des revendications 6 à 7, en tant que biocapteurs ou bioconducteurs.

11. Réactif chimique apte à être adsorbé physiquement sur des nanotubes de carbone, **caractérisé en ce qu'**il présente la formule générale **H-E-L,** dans laquelle :
- **H** représente un groupe hydrophile, sélectionné parmi les groupes chargés positivement ou négativement ; des ligands ou analogues de macromolécules biologiques ; des complexes organométalliques interagissant avec des acides aminés ou des acides nucléiques et dont les ligands sont éventuellement fonctionnalisés par des groupements alkyles de liaison à E ;
- **E** représente un bras espaceur, sélectionné parmi des chaînons carbonés en C₁-C₁₀, éventuellement substitués par des groupes alkyles, présentant ou non des insaturations ou des motifs poly-oxyéthylène pouvant présenter ou non en milieu de chaîne des groupes phosphate, tels que : 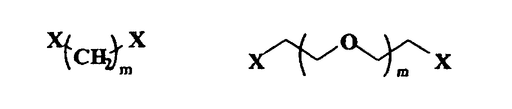 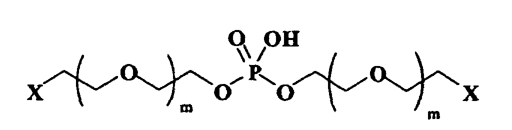 dans lesquels :
m représente un nombre entier de 1 à 10,
X représente O, NHCO, OCO, COO, CONH, S, CH₂ ou NH et constitue aux extrémités desdits chaînons carbonés des fonctions organiques d'accrochage du type esters, amides, éthers, thioéthers ;
- **L** représente un groupe aromatique de formule Ar₁ ou de formule Ar₂: 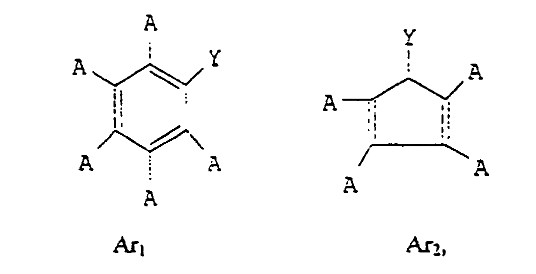 dans lesquelles :
A représente un atome d'hydrogène, l'un des groupes- suivants : alkyle, CF₃, NO₂, NH₂, OH, O-alkyle, S-alkyle, COOH, halogène, un cycle aromatique ou un hétérocycle aromatique en C₄-C₆, lesdits cycles étant éventuellement poly-substitués par des groupes électrodonneurs de type alkyle ou électroattracteurs de type CF₃ ou halogénures ; et
Y représente une liaison à E.

12. Réactif chimique selon la revendication 11, **caractérisé en ce qu'**il présente l'une des structures suivantes :

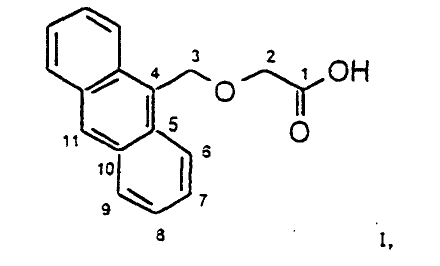

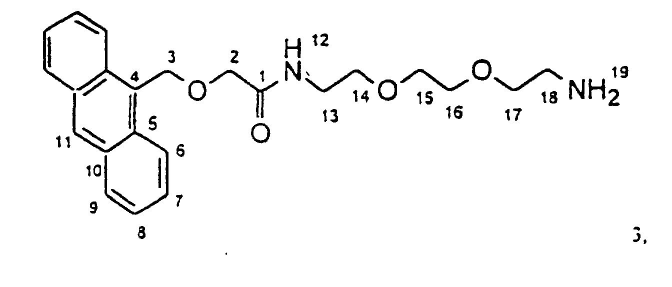

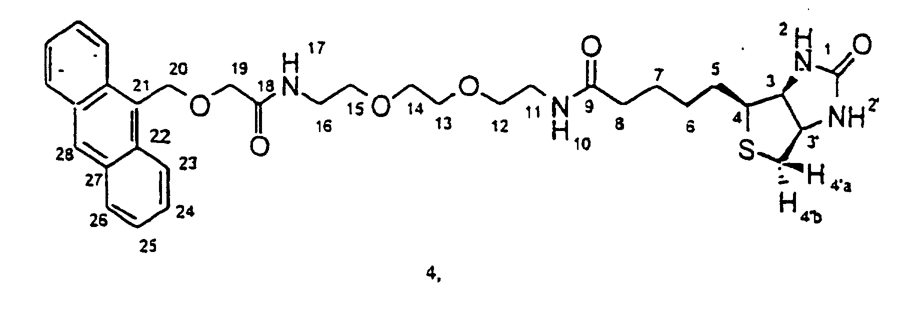

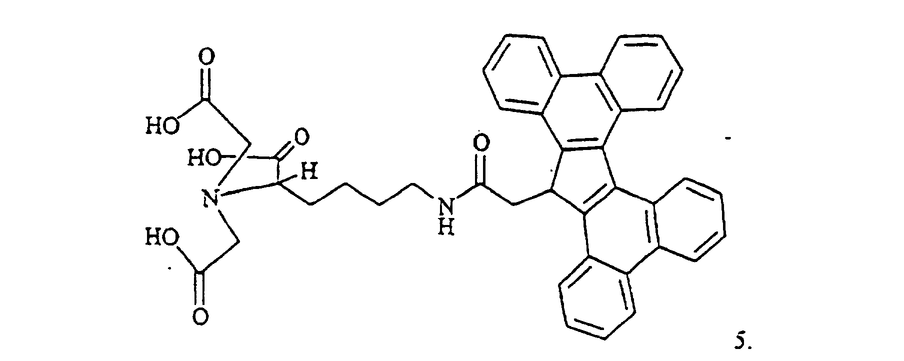

13. Réactif chirnique selon la revendication 11, **caractérisé en ce que** H est sélectionné parmi les complexes organbmétalliques suivants : 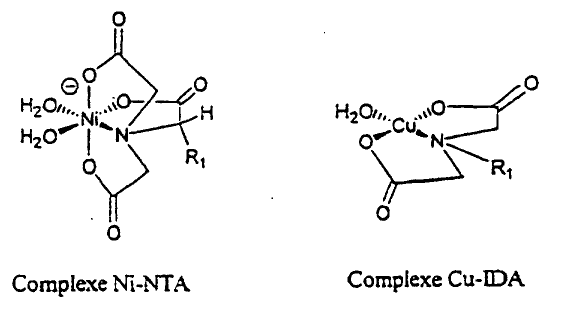 avec R₁ = groupe organique de liaison à E

## Patentansprüche

1. Verfahren zur Fixierung und Selbstorganisation von biologischen Makromolekülen, **dadurch gekennzeichnet, dass** es die Inkubation ohne Rühren eines biologischen Makromoleküls in Lösung mit Kohlenstoff-Nanoröhren, die an ihren Enden verschlossen sind, unter zweckdienlichen Temperaturund pH-Bedingungen für mindestens 15 Minuten umfasst.

2. Verfahren nach Anspruch 1, **dadurch gekennzeichnet, dass** die biologischen Makromoleküle insbesondere lösliche, Membran-, Transmembran-Proteine, Enzyme, Antikörper, Antikörperfragmente oder Nukleinsäuren sind.

3. Verfahren nach Anspruch 1 oder Anspruch 2, **dadurch gekennzeichnet, dass** die Kohlenstoff-Nanoröhren durch physikalische Adsorption eines chemischen Reagenzes der allgemeinen Formel **H-E-L** an ihrer Oberfläche funktionalisiert sind,
worin:
- **H** ein hydrophile Gruppe darstellt, die aus positiv oder negativ geladenen Gruppen; Liganden oder Analoga biologischer Makromoleküle; metallorganischen Komplexen, die mit den Aminosäuren oder den Nukleinsäuren wechselwirken und deren Liganden gegebenenfalls durch Alkylgruppierungen zur Bindung an **E** funktionalisiert sind, ausgewählt ist;
- **E** einen Spacer darstellt, ausgewählt aus C₁-C₁₀-Kohlenstoffketten, gegebenenfalls substituiert durch Alkylgruppen, enthaltend Ungesättigtheiten oder nicht, oder Polyoxyethylengruppierungen, die in der Kettenmitte Phosphatgruppen aufweisen können oder nicht, wie zum Beispiel: 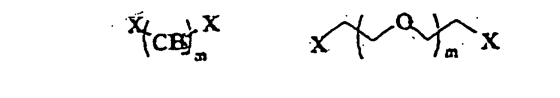 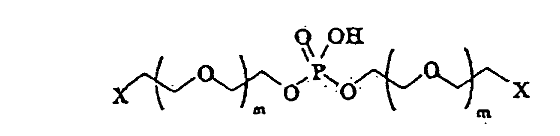 in denen:
m eine ganze Zahl zwischen 1 und 10 darstellt,
X für O, NHCO, OCO, COO, CONH, S, CH₂ oder NH steht und an den Enden der genannten Kohlenstoffketten organische Verankerungsfunktionen des Ester-, Amid-, Ether-, Thioether-Typs bildet;
- **L** eine Lipidgruppierung mit einer oder mehreren C₁₂-C₂₀-Ketten variabler Länge darstellt, die Unsättigungen oder auch nicht aufweist; eine aromatische Gruppe der Formel Ar₁ oder der Formel Ar₂ darstellt: 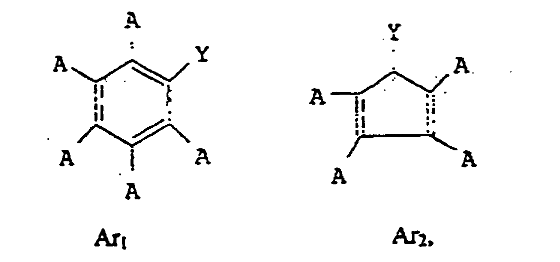 worin:
A ein Wasserstoffatom, eine der folgenden Gruppierungen: Alkyl, CF₃, NO₂, NH₂, OH, O-Alkyl, S-Alkyl, COOH, Halogen, ein aromatischer Ring oder ein aromatischer Heterocyclus mit C₄-C₆, gegebenenfalls mehrfach substituiert durch Elektronendonorgruppen des Alkyltyps oder Elektronenakzeptorgruppen des Typs CF₃ oder Halogenide; und
Y eine Bindung an E darstellt.

4. Verfahren nach einem der Ansprüche 1 bis 3, **dadurch gekennzeichnet, dass** die Lösung durch ein Lösungsmittel zur Solubilisierung der biologischen Makromoleküle, das wässrig oder wässrig-alkoholisch ist und gegebenenfalls mindestens ein Detergenz enthält, gebildet wird.

5. Verfahren nach einem der Ansprüche 1 bis 4, **dadurch gekennzeichnet, dass** die Inkubationsbedingungen vorzugsweise die Folgenden sind: Inkubation bei Umgebungstemperatur für 15 Minuten bis 48 Stunden bei einem pH zwischen 5,5 und 8,5.

6. Bionanomaterialien, **dadurch gekennzeichnet, dass** sie aus Kohlenstoff-Nanoröhren bestehend, auf denen die biologischen Makromoleküle in kristalliner Form selbstorganisiert sind.

7. Bionanomaterialien nach Anspruch 6, **dadurch gekennzeichnet, dass** sie mit Hilfe eines Verfahrens nach einem der Ansprüche 1 bis 5 erhalten werden.

8. Verwendung der Bionanomaterialien nach einem der Ansprüche 6 bis 7 zur Strukturuntersuchung biologischer Makromoleküle, die mit ihnen assoziiert sind.

9. Verwendung der Biomaterialien nach einem der Ansprüche 6 bis 7 als biologisches Reagenz.

10. Verwendung der Bionanomaterialien nach einem der Ansprüche 6 bis 7 als Biosensoren oder Bioleiter.

11. Chemisches Reagenz, das geeignet ist, physikalisch an den Kohlenstoff-Nanoröhren adsorbiert zu werden, **dadurch gekennzeichnet, dass** es die allgemeine Formel **H-E-L** aufweist,
in der:
- **H** ein hydrophile Gruppe darstellt, die aus positiv oder negativ geladenen Gruppen; Liganden oder Analoga biologischer Makromoleküle; metallorganischen Komplexen, die mit den Aminosäuren oder den Nukleinsäuren wechselwirken und deren Liganden gegebenenfalls durch Alkylgruppierungen zur Bindung an E funktionalisiert sind, ausgewählt ist;
- **E** einen Spacer darstellt, ausgewählt aus C₁-C₁₀-Kohlenstoffketten, gegebenenfalls substituiert durch Alkylgruppen, enthaltend Ungesättigtheiten oder nicht, oder Polyoxyethylengruppierungen, die in der Kettenmitte Phosphatgruppen aufweisen können oder nicht, wie zum Beispiel: 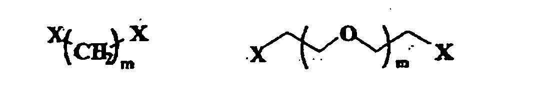 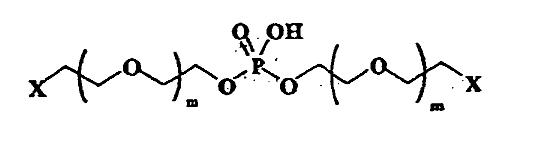 in denen:
m eine ganze Zahl zwischen 1 und 10 darstellt,
X für O, NHCO, OCO, COO, CONH, S, CH₂ oder NH steht und an den Enden der genannten Kohlenstoffketten organische Verankerungsfunktionen des Ester-, Amid-, Ether-, Thioether-Typs bildet;
- **L** eine aromatische Gruppe der Formel Ar₁ oder der Formel Ar₂ darstellt: 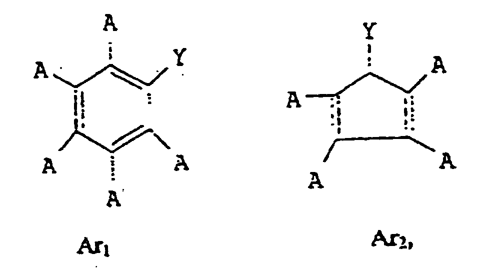 worin:
A ein Wasserstoffatom, eine der folgenden Gruppierungen: Alkyl, CF₃, NO₂, NH₂, OH, O-Alkyl, S-Alkyl, COOH, Halogen, ein aromatischer Ring oder ein aromatischer Heterocyclus mit C₄-C₆, gegebenenfalls mehrfach substituiert durch Elektronendonorgruppen des Alkyltyps oder Elektronenakzeptorgruppen des Typs CF₃ oder Halogenide; und
Y eine Bindung an E darstellt.

12. Chemisches Reagenz nach Anspruch 11, **dadurch gekennzeichnet, dass** es eine der folgenden Strukturen aufweist:

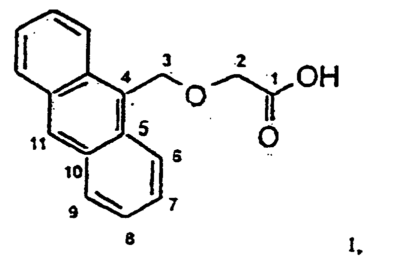

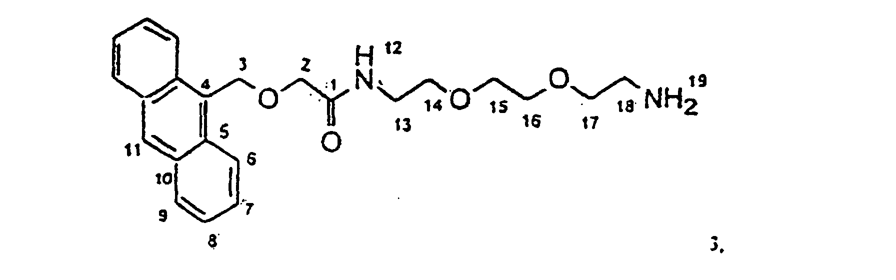

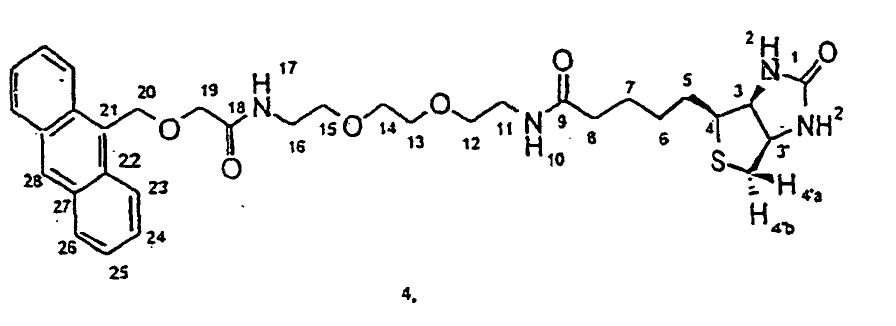

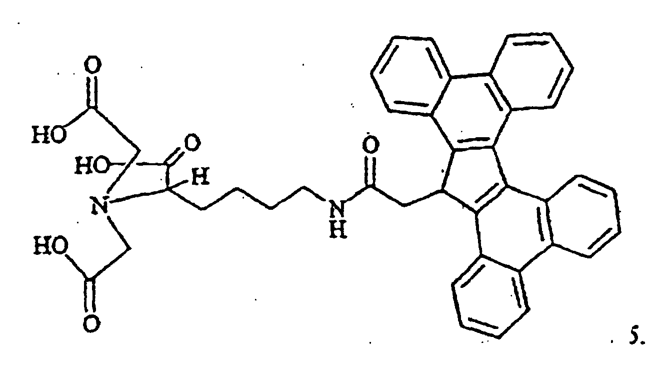

13. Chemisches Reagenz nach Anspruch 11, **dadurch gekennzeichnet, dass H** unter den folgenden metallorganischen Komplexen ausgewählt ist: 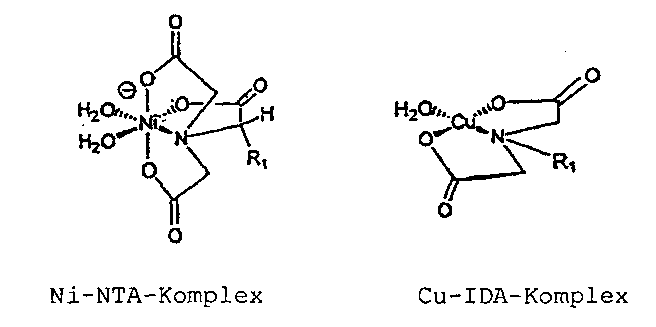 wobei R₁ = organische Gruppe zur Bindung an E.

## Claims

1. Method for the attachment and self-organization of biological macromolecules, **characterized in that** it comprises the incubation, without stirring, for at least 15 minutes, of a biological macromolecule in solution with nanotubes of carbon closed at their ends, under suitable temperature and pH conditions.

2. Method according to Claim 1, **characterized in that** the said biological macromolecules are in particular soluble, membrane or transmembrane proteins, enzymes, antibodies, antibody fragments or nucleic acids.

3. Method according to Claim 1 or Claim 2, **characterized in that** the said nanotubes of carbon are functionalized by physical adsorption, at their surface, of a chemical reagent of general formula **H-E-L**,
in which:
- **H** represents a hydrophilic group selected from the positively or negatively charged groups; ligands or analogues of biological macromolecules; organometallic complexes interacting with amino acids or nucleic acids and whose ligands are optionally functionalized with alkyl groups for bonding to **E**;
- **E** represents a spacer arm, selected from C₁-C₁₀ carbon chains, optionally substituted with alkyl groups, having unsaturations or otherwise or polyoxyethylene units which may have or otherwise in the middle of the chain phosphate groups, such as: 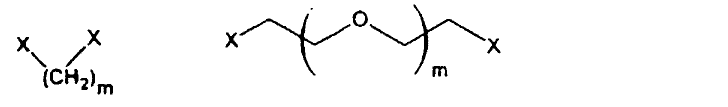 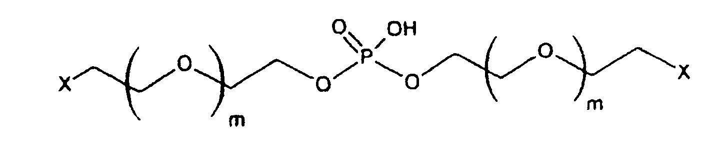 in which:
m represents an integer from 1 to 10,
X represents O, NHCO, OCO, COO, CONH, S, CH₂ or NH and constitutes, at the ends of the said carbon chains, organic coupling functions of the ester, amide, ether or thioether type;
- **L** represents a lipid unit with one or more chains of variable length in C₁₂-C₂₀ having unsaturations or otherwise; an aromatic group of formula Ar₁ or of formula Ar₂: 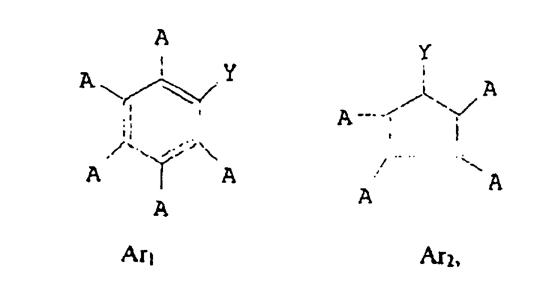 in which:
A represents a hydrogen atom, one of the following groups: alkyl, CF₃, NO₂, NH₂, OH, O-alkyl, S-alkyl, COOH, halogen, an aromatic ring or a C₄-C₆ aromatic heterocycle, optionally polysubstituted with electron-donating groups of the alkyl type or electron-attracting groups of the CF₃ or halide type; and
Y represents a bond with E.

4. Method according to any one of Claims 1 to 3, **characterized in that** said solution is constituted of a solvent for solubilizing said biological macromolecules, which is aqueous or hydroalcoholic and which optionally contains at least one detergent.

5. Method according to any one of Claims 1 to 4, **characterized in that** the incubation conditions are preferably the following: incubation at room temperature, for 15 minutes to 48 hours, at a pH of between 5.5 and 8.5.

6. Bionanomaterials, **characterized in that** they are constituted of nanotubes of carbon, on which biological macromolecules are self-organized in a crystalline form.

7. Bionanomaterials according to Claim 6, **characterized in that** they are obtained with the aid of a method according to any one of Claims 1 to 5.

8. Use of the bionanomaterials according to any one of Claims 6 to 7, for the structural study of the biological macromolecules which are associated with them.

9. Use of the bionanomaterials according to any one of Claims 6 to 7, as a biological reagent.

10. Use of the bionanomaterials according to any one of Claims 6 to 7, as biosensors or bioconductors.

11. Chemical reagent capable of being physically adsorbed on nanotubes of carbon, **characterized in that** it has the general formula **H-E-L,** in which:
- **H** represents a hydrophilic group selected from the positively or negatively charged groups; ligands or analogues of biological macromolecules; organometallic complexes interacting with amino acids or nucleic acids and whose ligands are optionally functionalized with alkyl groups for bonding to **E**;
- **E** represents a spacer arm, selected from C₁-C₁₀ carbon chains, optionally substituted with alkyl groups, having unsaturations or otherwise or polyoxyethylene units which may have or otherwise in the middle of the chain phosphate groups, such as: 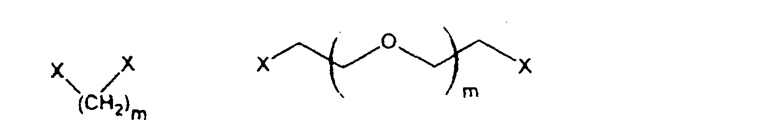 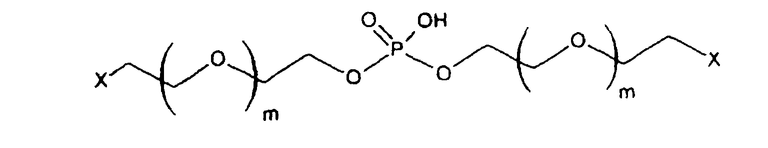 in which:
m represents an integer from 1 to 10,
X represents O, NHCO, OCO, COO, CONH, S, CH₂ or NH and constitutes, at the ends of the said carbon chains, organic coupling functions of the ester, amide, ether or thioether type;
- **L** represents an aromatic group of formula Ar₁ or of formula Ar₂: 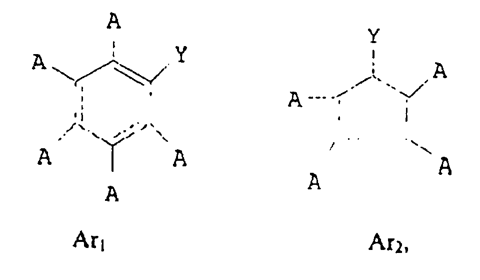 in which:
A represents a hydrogen atom, one of the following groups: alkyl, CF₃, NO₂, NH₂, OH, O-alkyl, S-alkyl, COOH, halogen, an aromatic ring or a C₄-C₆ aromatic heterocycle, said rings being optionally polysubstituted with electron-donating groups of the alkyl type or electron-attracting groups of the CF₃ or halide type; and
Y represents a bond with E.

12. Chemical reagent according to Claim 11, **characterized in that** it has one of the following structures:

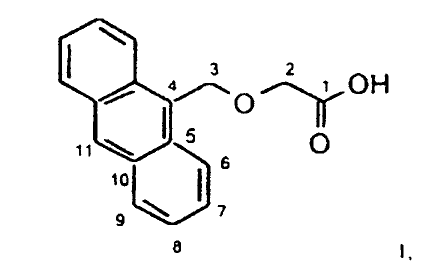

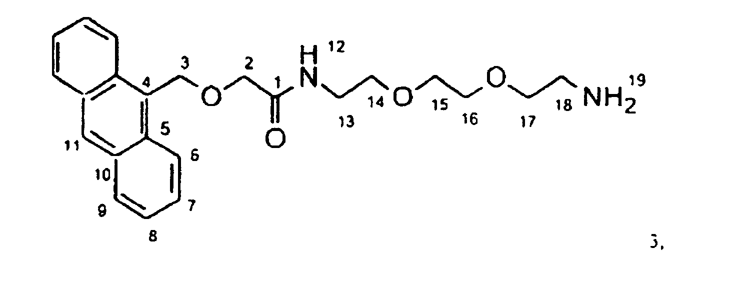

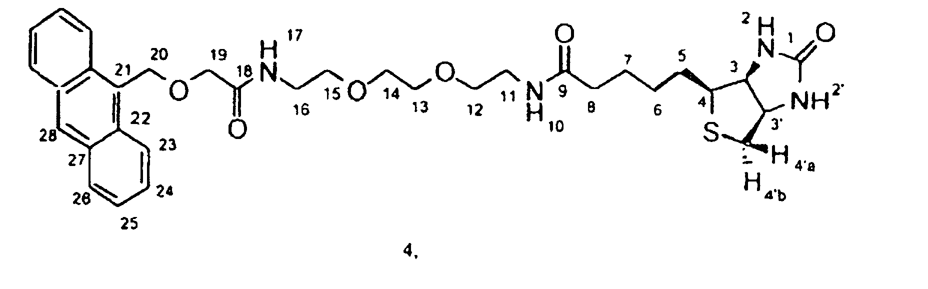

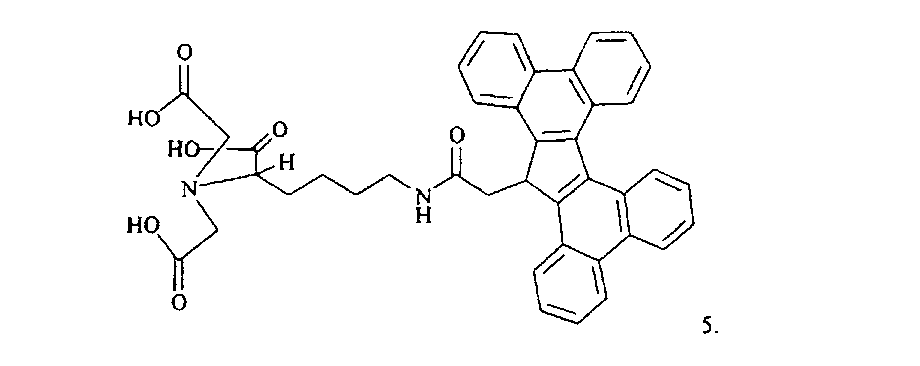

13. Chemical reagent according to Claim 11, **characterized in that** H is selected from the following organometallic complexes: 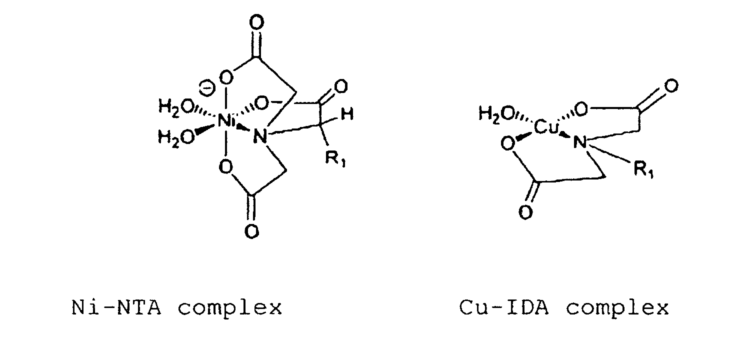 with R₁ = organic group for bonding to E.
